# EUROPEAN PATENT APPLICATION

(11) **EP 4 563 680 A1**
(43) Date of publication of application: **04.06.2025**
(21) Application number: 23212764.7
(22) Date of filing: 28.11.2023
(51) Int. Cl.: C11D 1/12, C11D 1/94, A61K 8/02, A61K 8/44, A61K 8/46

(54) **SOLID PERSONAL CARE OR DETERGENT COMPOSITION**

(71) Applicant: Dalli-Werke GmbH & Co. KG, 52224 Stolberg (DE)
(72) Inventor: REHFELDT, Iris, 67316 Carlsberg (DE); SCHARRER, Andreas, 68623 Lampertheim (DE); NICKEL, Janine, 67360 Lingenfeld (DE); THIEL, Rolf, 66123 Saarbrücken (DE); WIERCZIMOK, Dirk, 52076 Aachen (DE); PISKE, Tom, 52064 Aachen (DE)
(74) Representative: f & e patent

(57) **Abstract**

The present invention refers to new solid compositions comprising a combination of (a) acyl alkyl isethionate surfactant(s) and (b) amphoteric surfactants in a ratio of (a) to (b) from 2:1 to 1:2 in a total amount of from 50 to less than 100 wt.%, suitable for preparing aqueous personal care or cleaning detergent compositions by adding said solid composition to a predetermined amount of water.

## Description

The present invention refers to new solid compositions comprising a combination of acyl alkyl isethionate surfactant(s) and amphoteric surfactants suitable for preparing aqueous personal care or cleaning detergent compositions by adding said solid composition to a predetermined amount of water.

Many commonly available detergent or personal care compositions are in the form of viscous liquid compositions. However, these compositions are heavy and often contain large volumes of water. It is, however, expensive and environmentally unfriendly to transport large volumes of water in those compositions.

Solid detergent formulations offer significant advantages over liquid compositions. They are more compact and therefore easy to transport, they can be stored easier, and they are less susceptible for germination. In addition - if pre-dosed - a user typically only uses the amount needed and thus there is a reduction of wasted material.

Personal care or detergent compositions known in the art comprise mainly sulfate-surfactant based. These provide good foam or lather properties and can be rinsed well. However, a disadvantage of high-sulfate surfactant compositions is that sulfates are known to be degreasing and may feel harsh on the skin or the hair.

EP 2 794 830 A describes solid shampoo compositions including as a main ingredient acyl alkyl isethionates, and further comprising minor amounts of other surfactants. WO 2019/023332 A1 describes powdered shampoo compositions comprising sodium lauryl methyl isethionate as a primary surfactant besides 5 to 30 wt.% of a thickening agent. DE 10 2020 215078 A1 refers to powder compositions comprising 40 to 80 wt.% of at least one anionic surfactant and 10 to 30 wt.% of an amphoteric or zwitterionic surfactant.

It was an object of the present invention to provide solid compositions allowing to prepare aqueous liquid to gelled detergent or personal care compositions by adding the solid composition to water, wherein said compositions includes a low number of ingredients, in particular a low amount of conventional gelling or thickening polymeric components.

According to the present invention there is provided a solid personal care or detergent composition comprising a combination of (a) at least one acyl alkyl isethionate surfactant and (b) at least one amphoteric surfactant, in a ratio (a) to (b) of from 2:1 to 1:2, wherein each of said surfactants (a) and (b) independently is/are present in the solid composition in a total amount of from 20 to 60 wt.% based on the total amount of the solid composition, however, the total amount of said surfactants (a) and (b) together ranges from 45 to less than 100 wt.%.

The composition of the present invention is solid at room temperature (22±3°C), thus, preferably the ingredients of the inventive composition are all solid at room temperature. If one of the ingredients is not solid at room temperature, it is preferred that said ingredient is incorporated in small amounts (total content of liquid ingredients less than 10 wt.% of the total composition), preferably applied to one of the other solid ingredients of the composition, so that the total composition remains solid. The term "solid" is used with reference to normal atmospheric conditions (i.e. at a pressure of 1 atmosphere and 22±3°C). It is particularly preferred that both surfactant types (a) and (b) as such are also solid at a temperature of up to 40°C.

### Isethionate surfactant(s) (a)

Surfactants of type (a) comprises one or more compounds of formula (I): 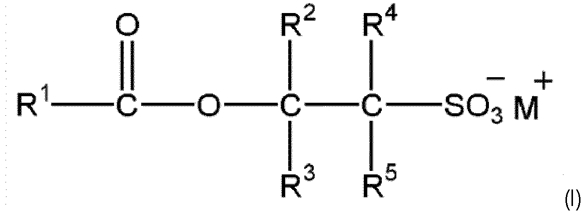 wherein R¹ represents a C₄₋₃₆ substituted or unsubstituted hydrocarbyl group; each of R², R³, R⁴ and R⁵ independently represents a hydrogen atom or a C₁₋₄ alkyl group and wherein at least one of R², R³, R⁴ and R⁵ is not hydrogen; and M+ represents a cation.

Preferably R¹ is selected from a substituted or unsubstituted alkyl, alkenyl, aryl or alkylaryl group. More preferably R¹ is selected from a substituted or unsubstituted alkyl or alkenyl group. Most preferably R¹ is an unsubstituted alkyl or alkenyl group, especially an unsubstituted alkyl group.

Preferably R¹ represents a C₄₋₂₄ or C₅₋₂₂ alkyl group, preferably a C₇₋₂₀ alkyl group, more preferably a C₈₋₁₈ alkyl group, most preferably a C₈₋₁₆ or a C₁₀₋₁₆ alkyl group.

Preferably R² represents a C₁₋₄ alkyl group or hydrogen, suitably a C₁₋₄ alkyl group in which a propyl or butyl group, when present, is straight-chained. Preferably R² represents an n-propyl, ethyl or, most preferably, a methyl group.

Preferably R³ represents a hydrogen atom.

Preferably one of R⁴ and R⁵ represents a hydrogen atom and the other represents a hydrogen atom or a C₁₋₄ alkyl group. Preferably one of R⁴ and R⁵ represents a hydrogen atom or a C₁₋₄ alkyl group in which a propyl or butyl group is straight-chain. Preferably one of R⁴ and R⁵ represents an n-propyl, ethyl or methyl group or, most preferably, a hydrogen atom. Most preferably both R⁴ and R⁵ represent hydrogen atoms, if R² is an alkyl group.

It is preferred that either one of R² or R⁴ or R⁵ is an alkyl group, preferably n-propyl, ethyl or methyl, more preferred ethyl or methyl, most preferred methyl, wherein the others are hydrogen. The presence of a short alkyl group, in particular a methyl group increases the water solubility of the compound noticeably.

The composition of the present invention may include a mixture of more than one compound of formula (I). For example, an isomeric mixture of compounds of formula (I) may be present. Such a mixture may include, for example a compound in which R² is alkyl (suitably methyl) and R³, R⁴ and R⁵ are all hydrogen and a compound in which R⁵ is an alkyl (suitably methyl) and R², R³ and R⁴ are all hydrogen. Preferably M+ represents an optionally substituted ammonium cation or, most preferably, a metal cation. Suitable ammonium cations include NH₄⁺ and the cation of monoethanolamine or triethanolamine. Suitable metal cations include alkali metal cations, for example sodium, lithium and potassium cations, and alkaline earth metal cations, for example calcium and magnesium cations. Preferably M+ represents a potassium or sodium cation. Most preferably M+ represents a sodium cation. The skilled person will appreciate that when M+ is a divalent metal cation two moles of anion will be present for each mole of cation.

R¹ may be an alkyl group or an alkenyl group. Preferably R¹ is an alkyl group. In some embodiments surfactants (a) of the present invention may comprise a mixture of fatty acid chains to form a mixture of compounds of formula (I) in which R¹ may be different.

R1 is preferably the residue of a fatty acid. Fatty acids obtained from natural oils often include mixtures of fatty acids. For example, the fatty acid obtained from coconut oil contains a mixture of fatty acids including lauric acid, myristic acid, palmitic acid, caprylic acid, and stearic and oleic acid.

R¹ may include the residue of one or more naturally occurring fatty acids and/or of one or more synthetic fatty acids. In some preferred embodiments R consists essentially of the residue of a single fatty acid.

Examples of carboxylic acids from which R¹ may be derived include coco acid, butyric acid, hexanoic acid, caproic acid, caprylic acid, capric acid, lauric acid, myristic acid, palmitic acid, palmitoleic acid, stearic acid, oleic acid, linoleic acid, arachidic acid, gadoleic acid, arachidonic acid, eicosapentanoic acid, behinic acid, eruic acid, docosahexanoic lignoceric acid, naturally occurring fatty acids such as those obtained from coconut oil, tallow, palm kernel oil, butterfat, palm oil, olive oil, corn oil, linseed oil, peanut oil, fish oil and rapeseed oil; synthetic fatty acids made as chains of a single length or a selected distribution of chain lengths; and mixtures thereof. Most preferably R¹ comprises the residue of lauric acid, that is a saturated fatty acid having 12 carbon atoms or the residue of mixed fatty acids derived from coconut oil. If the fatty acid residue R¹ is too long, water solubility of the compound decreases, thus a fatty acid with C₄₋₂₄ as defined above with reference to formula (I) is preferred.

In preferred embodiments the compound of formula (I) is the reaction product of sodium methyl isethionate and a fatty acid, that is a compound of formula R¹COOCHR²CHR⁴S0₃"M⁺ in which one of R² and R⁴ is methyl and the other is hydrogen. Mixtures of these isomers may be present. The composition may comprise a mixture of isomers, that is a compound of formula R¹COOCH₂CHR⁴S0₃"M⁺ in which R⁴ is alkyl (preferably methyl) and a compound of formula R¹COOCHR²CH₂S0₃"M⁺ in which R² is alkyl (preferably methyl). For example, the composition of the present invention may comprise one or more of sodium lauroyl methyl isethionate, sodium cocoyl methyl isethionate and sodium oleoyl methyl isethionate.

Most preferably the composition of the present invention comprises at least sodium lauroyl methyl isethionate and/or sodium cocoyl methyl isethionate. The presence of at least sodium lauroyl methyl isethionate is especially preferred.

The isethionate surfactant (a) according to the invention preferably is used and included into the composition in form of a powder, in particular a powder having a particle size in the range of from 0,01 µm to 3 mm, preferably from 0,05 µm to 2 mm, even more preferred from 0,1 to 1000 µm, including the lower limit of e.g. from 1 µm, 5 µm, 10 µm or 50 µm. It is preferred that less than 10 % by number of the particles have a particle size below the lower mentioned limit (defined by sieve analysis), more preferred less than 5 %, even more preferred less than 2 % or even less than 1 %, less than 0,5 % or less than 0,2 % by number. It is further preferred that the isethionate powder has a maximum particle size of 3 mm, preferably 2 mm, more preferred 1 mm, which means that the powder doesn't comprise particles having a particle size above 3 mm, or above 2 mm or above 1 mm to provide good dissolving properties to the solid composition of the present invention. For obtaining particles with a desired particle size distribution the particles can be ground and passed through an according sieve after grinding. The particle size range can be determined by common methods suitable for determining the volume mean particle size. However, it should be noted that the "minimum particle size" and the "maximum particle size" is meant to be an absolute value, which can be determined e.g. by sieve analysis, not an average, like it is obtained by laser diffraction analysis. In a particular preferred embodiment the particle size is "cut off" at the mentioned size limit. The isethionate surfactant suitable for the present invention has a good water solubility (is readily water dissolvable), however, if the particle size is too large the particle might not dissolve very fast, because at its surface a "gel film" is formed preventing further water from entering the particle. If the particle size is too small, the power might be too dusty, can be less handled and can remain in the package when the solid composition is added to water.

### Amphoteric surfactant(s) (b)

According to the present invention the composition comprises one or more amphoteric surfactant(s) (b) in addition to the surfactant(s) (a).

Suitable amphoteric surfactants (b) include those based on fatty nitrogen derivates and those based on betaines. Preferred are betaine(s); sultaine(s), amphodiproprionate(s) and amphoacetate(s).

Suitable amphoteric (or zwitterionic) surfactants may be selected from betaines, for example alkyl betaines, alkylamidopropyl betaines, alkylamidopropyl hydroxy sultaines, alkylampho-acetates, alkylamphodiacetates, alkylamphopropionates, alkylamphodipropionates, alkyliminodipropionates and alkyliminodiacetate.

Amphoteric or zwitterionic surfactants for use in the compositions of the present invention may include those which have an alkyl or alkenyl group of 7 to 22 carbon atoms and comply with a structural formula (II): 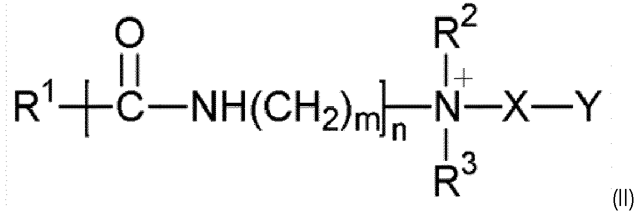 where R¹ is alkyl or alkenyl of 7 to 22 carbon atoms; R² and R³ are each independently alkyl, hydroxyalkyl or carboxyalkyl of 1 to 6 carbon atoms; m is 2 to 4; n is 0 or 1; X is alkylene of 1 to 6 carbon atoms optionally substituted with hydroxyl; and Y is -C0₂ or -S0₃. Amphoteric or zwitterionic surfactants may include simple betaines of formula: 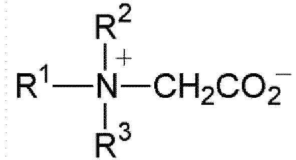 and amido betaines of formula: 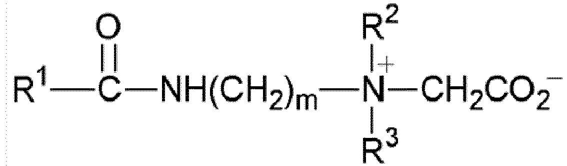 wherein m is 2 or 3.

In both formulae R¹, R² and R³ are as defined previously. R¹ may, in particular, be a mixture of C₁₂ and C₁₄ alkyl groups derived from coconut so that at least half, preferably at least % of the groups R¹ have 10 to 14 carbon atoms. R² and R³ are preferably methyl.

Amphoteric or zwitterionic surfactants may include sulfobetaines of formula: 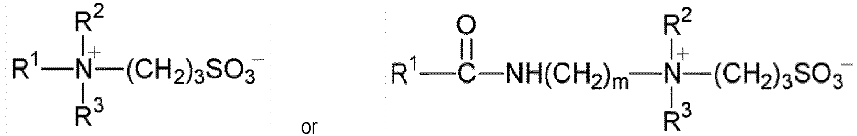
wherein m is 2 or 3, or variants of these in which -(CH₂)3S0₃" is replaced by 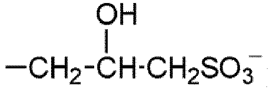
wherein R¹, R² and R³ in these formulae are as defined previously.

Amphoteric surfactant(s) (b) may also refer to amphoacetates and diamphoacetates. Amphoacetates generally conform to the following formula:

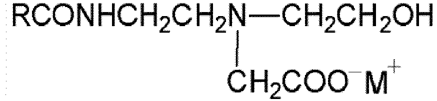

Diamphoacetates generally conform to the following formula: 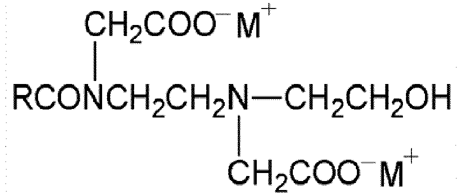 wherein R is an aliphatic group of 8 to 22 carbon atoms and M is a cation such as sodium, potassium, ammonium, or substituted ammonium.

Suitable acetate-based surfactants include lauroamphoacetate; alkyl amphoacetate; cocoampho(di)acetate; cocoamphoacetate; disodium cocoamphodiacetate; sodium cocoamphoacetate; disodium cocoamphodiacetate; disodium capryloamphodiacete; disodium lauroamphoacetate; sodium lauroamphoacetate and disodium wheatgermamphodiacetate.

Suitable betaine surfactants include alkylamido betaine; alkyl betaine, C_{12/14} alkyldimethyl betaine; cocoamidopropylbetaine; tallow bis(hydroxyethyl) betaine; hexadecyldimethylbetaine; cocodimethylbetaine; alkyl amido propyl sulfo betaine; alkyl dimethyl amine betaine; coco amido propyl dimethyl betaine; alkyl amido propyl dimethyl amine betaine; cocamidopropyl betaine; lauryl betaine; laurylamidopropl betaine, coco amido betaine, lauryl amido betaine, alkyl amino betaine; alkyl amido betaine; coco betaine; lauryl betaine; dimethicone propyl PG-betaine; oleyl betaine; N-alkyldimethyl betaine; coco biguamide derivative, C₈ amido betaine; C ₂ amido betaine; lauryl dimethyl betaine; alkylamide propyl betaine; amido betaine; alkyl betaine; cetyl betaine; oleamidopropyl betaine; isostearamidopropyl betaine; lauramidopropyl betaine; 2-alkyl-*N*-carboxymethyl-*N*-hydroxyethyl imidazolinium betaine; 2- alkyl-*N*-carboxyethyl-*N*-hydroxyethyl imidazolinium betaine; 2-alkyl-*N*-sodium carboxymethyl-*N*-carboxymethyl oxyethyl imidazolinium betaine; *N*-alkyl acid amidopropyl-*N*,*N*-dimethyl-*N*-(3- sulfopropyl)-ammonium-betaine; *N*-alkyl-*N,N*-dimethyl-*N*-(3-sulfopropyl)-ammonium-betaine; cocodimethyl betaine; apricotamidopropyl betaine; isostearamidopropyl betaine; myristamidopropyl betaine; palmitamidopropyl betaine; cocamidopropyl hydroxyl sultaine; undecylenamidopropyl betaine; cocoamidosulfobetaine; alkyl amido betaine; C_{12/14} alkyl amido propyl dimethyl amine betaine; lauryldimethyl betaine; ricinol amidobetaine; tallow aminobetaine.

It is preferred that the solid composition according to the invention comprises as at least one surfactant (b) at least cocoamidopropyl betaine. Surfactant (b) can also represented by cocoamidopropyl betaine, thus, containing this compound as the only surfactant (b).

The amphoteric surfactant (b) is preferably also added to the composition as a solid compound. Further it is preferred that said surfactant (b) is provided as a powder having a particle size distribution as mentioned above for the isethionate surfactant (a).

According to the present invention there is provided a solid composition comprising a total amount of at least 45 wt.% of surfactant(s) (a) and (b), wherein independently surfactant(s) of type (a) can be present in an amount of 20 to 60 wt.% and surfactant(s) of type (b) can be present in an amount of 20 to 60 wt.%, but the total amount of the surfactants (a) and (b) In the solid composition is less than 100 wt.%.

In this specification, unless otherwise indicated any amounts referred to relate to the amount of active component present in the composition.

Preferably the composition comprises one or more of surfactant(s) (a), in a total amount of at least 22 wt.% or at least 25 wt.%, for example at least 28 wt.% or at least 30 wt.% or up to 35 wt.%, at least 40 wt.% or up to 40 wt.%, at least 50 wt.% or up to 50 wt.%, or at least 55 wt.% or up to 55 wt.%, and up to 60 wt.%. The beforementioned values should be understood as including also all the ranges derivable from said values. In a particularly preferred embodiment the composition comprises surfactant (a) in a total amount of 25 to 40 wt.%. If more than one, e.g. two or three surfactants of type (a) (differing in formula I) are added to the composition the amount of the single surfactant may be lower than 20 wt.%, but the total amount of the surfactants should remain in the given ranges.

Preferably the composition comprises one or more of surfactant(s) (b), in a total amount of at least 22 wt.% or at least 25 wt.%, for example at least 28 wt.% or at least 30 wt.% or up to 35 wt.%, at least 40 wt.% or up to 40 wt.%, at least 50 wt.% or up to 50 wt.%, or at least 55 wt.% or up to 55 wt.%, and up to 60 wt.%. The beforementioned values should be understood as including also all the ranges derivable from said values. In a particularly preferred embodiment the composition comprises surfactant (b) in a total amount of 25 to 40 wt.%. If more than one, e.g. two or three surfactants of type (b) are added to the composition the amount of the single surfactant may be lower than 20 wt.%, but the total amount of the surfactants should remain in the given ranges.

Preferably the composition of the present invention comprises at least 50 wt.% or at least 55 wt.% as a total amount of surfactants (a) and (b), preferably at least 60 wt.%, at least 65 wt.% or at least 70 wt.%, at least 72 wt.% or at least 75 wt.%, at least 78 wt.% or even at least 80 wt.%. The composition can comprise up to 60 wt.%, up to 65 wt.% or up to 70 wt.%, up to 72 wt.%, or up to 75 wt.%, or up to 78 wt.%, up to 80 wt.%, or up to 85 wt.%, or up to 90 wt.% or up to 95 wt.%, or even up to 98 wt.%. The beforementioned values should be understood as including also all the ranges derivable from said values. In a particularly preferred embodiment the composition of the present invention comprises surfactants (a) and (b) in a total amount of from 50 to 75 wt.%, or even 55 to 65 wt.%.

Surfactant(s) of type (a) and surfactant(s) of type (b) are present in the composition of the invention in a ratio of from 2:1 to 1:2. This includes all ratios between these limits, e.g. a ratio of from 1,9:1 to 1:1,9, a ratio of from 1,8:1 to 1:1,8, a ratio of from 1,7:1 to 1:1,7, a ratio of from 1,6:1 to 1:1,6, a ratio of from 1,5:1 to 1:1,5, a ratio of from 1,4:1 to 1:1,4, a ratio of from 1,3:1 to 1:1,3, a ratio of from 1,2:1 to 1:1,2, a ratio of from 1,1:1 to 1:1,1 and a ratio of 1:1 and all the combinations of ranges as mentioned (e.g. 1,9:1 to 1:1,2). Further these ratios should be considered as including a ratio of (a) to (b) of e.g. 1,3:1, a ratio of 1,2:1, a ratio of 1,1:1 or a ratio of 1:1,1.

The solid composition of the present invention is provided as a powder, a granulate or a tablet, preferably in powder or granulate form. It is particularly preferred that the particle size of the particles in the solid composition according to the invention fulfills the same particle size requirements as defined above for surfactant (a). This means that either a powder is provided , wherein all the powder particles are within the defined size ranges, or a tablet is pressed from particles having the according size. If granules are provided, it is preferred that the granules have either a maximum particle size of 3 mm, or they are larger, but being prepared from (primary) particles having the according size(s). If tablets are provided, it might be suitable and preferred to include any ingredient into the tablet which supports the fast disintegration / decomposition of the tablet to particles of the above mentioned size.

The composition can be provided in pre-dosed portions, which can be added to a determined amount of water to prepare a ready-to-use aqueous composition. The composition is preferably a composition suitable to prepare an aqueous personal care, in particular a skin, hair or body care composition, bath or shower gel, or a detergent composition like a hard surface cleaning composition or a laundry composition, e.g. for clothes washed by hand by adding said composition to water.

Before the composition is added to water, the solid composition preferably comprises less than 5 wt.%, preferably less than 3 wt.%, more preferred less than 2 wt.%, and even more preferred less than 1 wt.% of water. If any other liquid component is included in the solid composition, it is preferred that the total amount of liquid in the solid composition is 10 wt.% or less. This amount refers to the total of all liquids included in the composition. It should be noted that during preparation the liquid content of the composition might be higher for a short period of time, however, if this is the case, the composition is dried then after addition of the liquid(s) to an amount of 10 wt.% or less to result in the solid composition of the present invention. Liquid components preferably are included into the composition by applying them to any of the solid ingredients.

### Additional ingredients:

The composition might comprise at least one further ingredient(s), in particular to provide compositions suitable for an intended application.

The composition can include e.g. any further surfactant(s) different from surfactant (a) and (b). Suitable additional surfactants (which means that they might be added to the composition in addition to surfactants (a) and (b)) may be selected from anionic surfactants, cationic surfactants and non-ionic surfactants. Preferred further surfactant(s) can be selected from anionic or nonionic surfactants.

### Anionic surfactant(s)

It might be of interest to include an anionic surfactant (c), preferably selected from sulfate, sulfonate, sulfoacetate, sulfosuccinate, or carbonate surfactants, in particular alkyl sulfate, alkyl sulfonate, alkyl benzene sulfonate, alkyl sulfoacetate, alkyl sulfosuccinate surfactants.

For each and any of the mentioned surfactants the alkyl chain independently can be selected from a C₆₋₂₄ alkyl chain, more preferred a C₈₋₂₂ alkyl chain, even more preferred a C₁₀₋₂₀ alkyl chain and even more preferred a C₁₂₋₁₈ alkyl chain, or a mixture of surfactants as defined before, having different alkyl chain length.

Suitable anionic surfactants for use in the composition of the present invention include salts of carboxylic acids, ethoxylated carboxylic acids, ester carboxylates and ethoxylated ester carboxylates and sarcosinates. Other suitable anionic surfactants include sulfates and sulfonates, for example alkyl sulfates, alkyl ether sulfates, alcohol sulfates, alcohol ether sulfates, a-olefin sulfonates, linear alkyl sulfonates; and phosphate esters. Suitable anionic surfactants may be selected from salts of fatty acids; alkali metal salts of mono- or dialkyl sulfates; mono- or dialkyl ether sulfates; lauryl ether sulfates; alkyl sulfonates; alkyl aryl sulfonates; primary alkane disulfonates; alkene sulfonates; hydroxyalkane sulfonates; alkyl glyceryl ether sulfonates; alpha-olefinsulfonates; alkyl phosphates; sulfonates of alkylphenolpolyglycol ethers; salts of alkyl sulfopolycarboxylic acid esters; alkyl sulfosucci nates and salts thereof, alkyl ether sulfosuccinates and salts thereof, non-acylated alkyl isethionates; fatty acid taurates; acyl taurates; products of condensation of fatty acids with oxy- and aminoalkanesulfonic acids; sulfated derivatives of fatty acids and polyglycols; alkyl and acyl sarcosinates; sulfoacetates; alkyl phosphates; alkyl phosphate esters; acyl lactates; alkanolamides of sulfated fatty acids and salts of lipoamino acids.

Exemplary salts of the above, where applicable, are the sodium, potassium, ammonium, monoethanolamine and triethanolamine salts.

Preferred additional anionic surfactants (c) for use in the present invention include alkyl glyceryl ether sulfonate, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium coco sulfate, ammonium lauroyl sulfate, sodium coco sulfate, sodium lauroyl sulfate, potassium coco sulfate, potassium lauryl sulfate, triethanolamine lauryl sulfate, triethanolamine lauryl sulfate, monoethanolamine coco sulfate, monoethanolamine lauryl sulfate, palm kernel oil sulfate, sodium tridecyl benzene sulfonate, sodium dodecyl benzene sulfonate, and combinations thereof.

Particularly preferred anionic surfactants for use herein include sodium cocoyl glutamate, sodium cocoyl isethionate, sodium lauryl sulfo acetate, alkyl sulfates, for example sodium lauryl sulfate or sodium coco sulfate and alkyl ether sulfates, for example sodium lauryl ether sulfate.

If present, it is preferred that surfactant(s) (c) is/are present in a total amount of from 0,2 to 10 wt.% of the total solid composition, preferably in an amount of from 0,5 to 8 wt.%, more preferred in an amount of from 1 to 6 wt.%.

It should be noted that any soap, i.e. fatty carboxylic acid salt(s) according to the present disclosure is not encompassed in the term "anionic surfactant".

### Nonionic surfactant(s)

As a further ingredient (d) at least one nonionic surfactant(s) can be present in the solid composition.

Suitable non-ionic surfactants for use herein include fatty alcohol ethoxylates and ethylene oxide/propylene oxide copolymer derived surfactants, sugar esters, especially sorbitan esters, alkyl polyglucosides, fatty acid ethoxylates or polyethylene glycol esters and partial esters, alkanolamides and amineoxides.

Especially preferred non-ionic surfactants for use herein include fatty acid alkanolamides, ethylene glycol stearate and ethylene glycol distearate.

Suitable non-ionic surface-active agents may be selected from reaction products of compounds having a hydrophobic group and a reactive hydrogen atom, for example aliphatic alcohols, acids, amides or alkyl phenols with alkylene oxides, especially ethylene oxide either alone or with propylene oxide (for example alkyl (C₆-C₂₂) phenol-ethylene oxide condensates, the condensation products of aliphatic (C₈ -C ₂₄) primary or secondary linear or branched alcohols with ethylene oxide, and products made by condensation of ethylene oxide with the reaction products of propylene oxide and ethylenediamine); long chain tertiary amine oxides, long chain tertiary phosphine oxides and dialkyl sulfoxides; alkyl amine oxides, alkyl amido amine oxides; alkyl tertiary phosphine oxides; alkoxyl alkyl amines; sorbitan; sorbitan esters; sorbitan ester alkoxylates; glycerol ester alkoxylates; sucrose esters; sugar amides, such as a polysaccharide amide; lactobionamides; and alkyl polysaccharide nonionic surfactants, for example alkylpolyglycosides. Preferred non-ionic surfactants are sucroglycerides and ethyoxylated fatty alcohols, especially those derived from lauryl, cetylstearyl, stearyl, cetyl and oleocetyl alcohols.

As suitable amide nonionic surfactant an amide surface active agent having the structure: 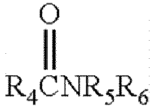
wherein R₄ is lauric acid, coconut acid, palmitic acid, myristic acid, behenic acid, babassu acid, isostearic acid, stearic acid, corn fatty acid, soy fatty acid, soy fatty acid, shea butter fatty acid, caprylic acid, capric acid, and mixtures thereof;
R₅ is -CH₂OH, -CH₂CH₂OH, -CH₂CH₂CH₂OH, -CH₂(CHOH)₄CH₂OH, -benzyl, and mixtures thereof; and
R₆ is -H, -CH₃, -CH₂OH, -CH₂CH₃, -CH₂CH₂OH, -CH₂CH₂CH₂OH, -CH₂(CHOH)₄CH₂OH, -benzyl, and mixtures thereof can be used.

Examples of preferred nonionic amide surfactant for use in the present compositions include cocamide monoethanolamine, cocamide diethanolamine; lauramide monoisopropylamide (MIPA), soyamide diisopropylamine (DIPA), and capryloyl/caproyl methyl glucamide, as well as mixtures of those materials.

If present, it is preferred that surfactant(s) (d) is/are present in a total amount of from 0,2 to 10 wt.% of the total solid composition, preferably in an amount of from 0,5 to 8 wt.%, more preferred in an amount of from 1 to 6 wt.%.

### Cationic surfactant(s)

As a further component (e) cationic surfactant(s) can be included in the composition. Suitable cationic surfactants for use herein are typically based on fatty amine derivates or phosphonium quaternary ions, and quaternary ammonium compounds.

Suitable cationic surfactants can include tertiary amine salts, mono alkyl trimethyl ammonium chloride, mono alkyl trimethyl ammonium methyl sulfate, dialkyl dimethyl ammonium chloride, dialkyl dimethyl ammonium methyl sulfate, trialkyl methyl ammonium chloride and trialkyl methyl ammonium methyl sulfate.

Some especially preferred cationic surfactants for use herein are monoalkyl quaternary ammonium compounds in which the alkyl chain length preferably is C₁₆ to C₂₂.

Examples of suitable cationic surfactants include quaternary ammonium compounds, particularly trimethyl quaternary compounds. Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e. g., bromide), acetate, citrate, lactate, glycolate, phosphate nitrate, sulfate, or alkylsulfate.

Further suitable cationic surfactants include those materials having the CTFA designations Quaternium-5, Quaternium-31 and Quaternium-18. Mixtures of any of the foregoing materials may also be suitable. A particularly useful cationic surfactant for use as a hair conditioning agent is cetyltrimethylammonium chloride.

Salts of primary, secondary, and tertiary fatty amines are also suitable cationic surfactants. The alkyl groups of such amines preferably have from 12 to 22 carbon atoms, and can be substituted or unsubstituted.

Useful cationic surfactants include amido substituted tertiary fatty amines, in particular tertiary amines having one C₁₂ to C₂₂ alkyl or alkenyl chain. Such amines include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachid amidopropyldiethylamine, arachidamidoethyldiethylamine, arachidamidoethyldimethylamine, diethylaminoethylstearamide.

Also useful are dimethylstearamine, dimethylsoyamine, soyamine, myristylamine, tridecylamine, ethylstearylamine, tallowpropane diamine, ethoxylated (with 5 moles of ethylene oxide) stearylamine, dihydroxyethylstearylamine, and arachidyl behenylamine.

These amines are typically used in combination with an acid to provide the cationic species. Suitable acids include L-glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid.

Further, the composition might comprise surfactants based on amino acids, like e.g. glycinates. Examples for glycinate surfactants include cocoamphocarboxyglycinate; tallowamphocarboxygycinate; capryloamphocarboxyglycinate, oleoamphocarboxyglycinate, bis-2-hydroxyethyl tallow glycinate; lauryl amphoglycinate; tallow polyamphoglycinate; coco amphoglycinate; oleic polyamphoglycinate; dihydroxyethyl tallow gycinate. These are very mild, and thus recommendable for skin and body care products.

If present, it is preferred that cationic surfactant(s) (e) is/are present in a total amount of from 0,2 to 10 wt.% of the total solid composition, preferably in an amount of from 0,5 to 8 wt.%, more preferred in an amount of from 1 to 6 wt.%.

### Cationic polymers

Suitable cationic polymers are preferably hair-conditioning polymers. Such polymers contain preferably quaternary amine groups. The cationic polymers can be homopolymers or copolymers, in which the quaternary nitrogen groups either are contained in polymer chains or backbones or preferably as substituents in one or more of the monomers. Monomers containing ammonium groups monomers can be copolymerized with non-cationic monomers. Suitable cationic monomers include unsaturated, radically polymerizable compounds, which have at least one cationic group, especially ammonium-substituted vinyl monomers, such as trialkylmethacryloxyalkyl ammonium groups, trialkylacryloxyalkyl ammonium groups, dialkyldiallyl ammonium groups and quaternary vinyl ammonium monomers with cyclic, cationic nitrogen-containing groups, such as pyridinium, imidazolium or quaternary pyrrolidone groups, e.g. alkylvinyl imidazolium, alkylvinylpyridinium, or alkylvinylpyrrolidone salts. The alkyl groups of these monomers are preferably lower alkyl groups, preferably C1- to C7-alkyl groups, especially preferably C1- to C3-alkyl groups.

The monomers containing the ammonium groups can be copolymerized with non-cationic monomers. Suitable comonomers are, for example, acrylamide, methacrylamide, alkylacrylamides, dialkylacrylamides, alkylmethacrylamides, dialkylmethacrylamides, alkylacrylates, alkylmethacrylates, vinyl caprolactone, vinylcaprolactam, vinyl pyrrolidone, vinyl esters, e.g. vinyl acetate, vinyl alcohols, propylene glycol or ethylene glycol. The alkyl groups of these monomers are preferably C1- to C7-alkyl groups, especially preferably C1- to C3-alkyl groups.

Suitable cationic polymers with quaternary amino groups are, for example, the polymers described in the CTFA Cosmetic Ingredient Dictionary under the trade name polyquaternium, for example methylvinylimidazolium chloride/vinyl pyrrolidone copolymer (polyquaternium-16) or quaternized vinyl pyrrolidone/dimethylaminoethylmethacrylate copolymer (polyquaternium-11).

Suitable cationic polymers, which are derived from natural polymers, include cationic derivatives of cellulose, starch or guar. Chitosan and chitosan derivative compounds are also suitable. Suitable polysaccharides have the general formula (V):

G-(O-B-N⁺R^{a}R^{b}R^{c})ₙX⁻ (V),

wherein G is a anhydroglucose residue, for example starch or cellulose anhydroglucose and n is a number greater than 0, preferably 1 to 40; O is the etherified hydroxy group of the cellulose polymer; B is a divalent group, for example, an alkylene, an oxyalkylene, a polyoxyalkylene or hydroxyalkylene; R^{a}, R^{b} and R^{c} are each, independently of each other, alkyl, aryl, alkylaryl, arylalkyl, alkoxyalkyl or alkoxyaryl with up to 18 carbon atoms respectively, wherein the total number of carbon atoms in R^{a}, R^{b} and R^{c} is at most 20; X- is a common counter anion and is preferably chloride. An example of a suitable cationic cellulose compound has the INCI name, polyquaternium-10. A further suitable cationic cellulose compound has the INCI name polyquaternium-24. A suitable cationic guar derivative compound has the INCI name guar hydroxypropyltrimonium chloride.

Further suitable cation-active hair-care compounds are cationically modified protein derivative compounds or cationically modified protein hydrolyzates and for example are known under the INCI name lauryidimonium hydroxylpropyl hydrolyzed wheat protein, lauryidimonium hydroxypropyl hydrolyzed caesin, lauryldimonium hydroxypropyl hydrolyzed collagen, lauryidimonium hydroxypropyl hydrolyzed keratin, lauryidimonium hydroxypropyl hydrolyzed silk, lauryidimonium hydroxypropyl hydrolyzed soy protein or hydroxypropyltrimonium hydrolyzed wheat, hydroxypropyltrimonium hydrolyzed casein, hydroypropyltrimonium hydrolyzed collagen, hydroxypropyltrimonium hydrolyzed keratin, hydroxypropyltrimonium hydrolyzed rice bran protein, hydroxypropyltrimonium hydrolyzed silk, hydroxypropyltrimonium hydrolyzed soy protein, hydroxypropyltrimonium hydroxlyzed vegetable protein.

### α-hydroxy acid

The composition can comprise further citric acid and/or citrate salt. For compositions comprising at least one of those, it is preferred that
(i) citric acid is present in an amount of from 0,5 to 20 wt.% of the total solid composition, preferably in an amount of from 1 to 15 wt.%, more preferred from 2 to 10 wt.% even more preferred from 3 to 8 or from 4 to 6 wt.% of the total solid composition and/or
(ii) citrate salt is present in an amount of from 0,5 to 20 wt.% of the total solid composition, preferably in an amount of from 1 to 15 wt.%, more preferred from 2 to 10 wt.% even more preferred from 3 to 8 wt.% or from 4 to 6 wt.% of the total solid composition.

It is however preferred that the total amount of a combination of citric acid and citrate salt in the total solid composition ranges from 1 to 30 wt.%, preferably 2 to 25 wt.%, more preferred 4 to 20 wt.%, even more preferred 5 to 15 wt.% or 6 to 10 wt.% of the total solid composition.

Instead of or in addition to the citric acid and/or citrate salt the composition can also comprise any other α-hydroxy acid like e.g. lactic acid, maleic acid, glycolic acid, mandelic acid, tartaric acid, or isocitric acid, wherein it is preferred that the total amount of the α-hydroxy acid(s) - except citric acid and citrate salt - in the total solid composition ranges from 0,5 to 30 wt.%, preferably 1 to 25 wt.%, more preferred 2 to 20 wt.%, even more preferred 2,5 to 15 wt.% or 3 to 10 wt.% of the total solid composition.

### Complexing/chelating agent

The composition can further comprise a complexing (chelating) agent separate from citric acid, citrate or any of the α-hydroxy acids. Said complexing agent is preferably selected from methylglycinediacetic acid (MGDA) or glutamic acid-N,N-diacetic acid (GLDA), ethylenediamine-N,N'-disuccinic acid, imminodisuccinic acid, diethylene triamine pentaacetic acid, ethylenediamine tetraacetic acid, (polymeric) phosphonate(s), e.g. diethylenetriamine pentamethylene phophonate, tetrahydroxypropyl ethylenediamine, triisobutylphosphate, EDTA, dicarboxymethyl alaninate, etidronate, polyphosphate, maleic acid/acrylic acid copolymer(s), each of them preferably in form of their sodium salts.

Preferred chelants are biodegradable chelants for example ethylenediamine-N,N'-disuccinic acid, methylglycinediacetic acid, glutamic acid-N,N-diacetic acid, imino disuccinic acid and anions and mixtures thereof.

Any complexing agent(s) preferably is/are present in a total amount of from 0,05 to 10 wt.%, preferably from 0,1 to 8 wt.%, more preferred from 0,5 to 6 wt.%.

### Preservative

The composition further can comprise a preservative agent, preferably selected from benzoic acid, benzoates like e.g. sodium benzoate, sorbates like e.g. potassium sorbate, sulfites, phenoxyethanol, sodium and zinc pyrithione, bronopol, iodopropynylbutylcarbamate, formaldehyde and formaldehyde releaser, glyoxal, glutaral and isothiazolinones like e.g. methylisothiazolinone, chloromethylisothiazolinone, benzisothiazolinone, octylisothiazolinone, and butylbenzisothiazolinone, wherein preferably at least a benzoate or a sorbate is present as at least one preservative agent, or these both are present in the solid composition.

The composition according to the present invention might comprise the preservative agent(s) independently in an amount of from 0,2 to 10 wt.% of the total solid composition, preferably in an amount of from 0,5 to 8 wt.%, more preferred from 0,8 to 6 wt.% even more preferred from 1 to 5 wt.% of the total solid composition. The total amount of preservative agents in the solid composition can be 0 to 15 wt.%, preferably 1 to 12 wt.%, 2 to 10 wt.% or 3 to 8 wt.% of the total composition.

### Hygroscopic compound

The composition can further comprise a total amount of 0 to 60 wt.% of at least one skin compatible humidity-uptaking (hygroscopic) compound, in particular any of C4- C5- or C6- sugars or dimers or oligomers thereof, preferably glucose, fructose, saccharose, sorbitol, erythritol, or any hygroscopic salt like e.g. sodium chloride or sodium sulfate.

The solid composition can comprise each of the at least one humidity-uptaking compound independently in an amount of from 0 to 30 wt.%, preferably 1 to 20 wt.%, more preferred 2 to 10 wt.%. The total amount of said agents in the total composition is within the same range.

### Oil / fat / wax

The compositions of the present invention may include an oil which is a fatty triglyceride liquid at room temperature (about 25 degrees centigrade). When used, this oil may constitute from about 0.1 wt.% to about 10 wt.%, such as from about 0.2 wt.% to about 5 wt.% or 0,5 wt.% to about 3 wt.% of the present composition. Examples include natural oil or mineral oil wherein natural oil compounds are preferred. Such natural oils can be obtained from any oil-comprising plant, fruit or seed.

The compositions of the present invention may also include from about 1 wt.% to about 10 wt.%, such as from about 2 wt.% to about 8 wt.% or from about 3 wt.% to about 5 wt.% of at least one fat (soft solid triglyceride), wax and/ or at least one fatty alcohol. The fatty alcohols can be linear or branched, saturated or unsaturated with from 10 to 30, for example, 12 to 26, carbon atoms in their alkyl chains. Non-limiting examples of such materials include myristyl alcohol, cetyl alcohol, stearyl alcohol, cetearyl alcohol, palmityl alcohol, oleyl alcohol, lauryl alcohol, coconut alcohol, palm alcohol, behenyl alcohol, arachidyl alcohol, and mixtures of those materials. Exemplary embodiments include cetearyl alcohol, cetyl alcohol, myristyl alcohol and stearyl alcohol, as well as mixtures of those materials.

Further, the composition of the invention may comprise any wax, preferably from a natural source. A wax comprises at least an ester of a fatty acid and a fatty alcohol, wherein it is preferred that the fatty acid and fatty alcohol compounds each have a chain length of at least 10 C atoms. Further, natural waxes commonly comprise free long chain fatty acids, fatty alcohols and or hydrocarbons. Preferred are waxes from animals or plants, e.g. bees wax, lanolin, china wax, spermaceti, jojoba oil, carnauba wax etc.

Emulsifiers, generally nonionic, may also be included in the compositions of the present invention in order to compatibilize the oily and aqueous components found in the compositions. Examples of nonionic emulsifiers which can be used in the present invention include sorbitan fatty acid esters (such as sorbitan monoleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan, tetra-2-ethylhexylate; glycerol or polyglycerol fatty acid esters (such as glycerol mono-cotton seed oil fatty acid ester, glycerol monoerucate, glycerol sesquioleate, glycerol monostearate, glycerol-a, a'-oleate pyroglutamate, and glycerol monostearate malate); propylene glycol fatty acid esters (such as propylene glycol monostearate); hardened castor oil derivatives; and glycerol alkyl ethers. Exemplary nonionic emulsifier materials include glyceryl derivatives, such as glyceryl stearate. Mixtures of those materials may be used.

The compositions of the present invention may also include any further conventional additives which are typically used in personal care (in particular hair, skin or body care) or in detergent compositions, at their conventional art-established levels, as long as those additives are compatible with the other components and do not negatively affect the present invention. Additives which may be used in various compositions include waxes, fatty acid esters of varying chain lengths, polymers, alcohols, polyhydric alcohols, extracts useful for providing fragrance, vitamins, hydrolyzed proteins and derivatives thereof, glycerin and derivatives thereof, anti-inflammatory agents, microbiocides, anti-fungals, antiseptics, antioxidants, UV absorbers, dyes and pigments, sunscreen active agents, oxidizers, pH balancing agents, moisturizers and the like, each of them approved for use in formulations for human use. In addition, additives such as EDTA, glutamic acid, glycerin, pantenol, and pH adjustment additives may be included. Mixtures of those materials may also be used. Solid components are particularly preferred.

Examples of antioxidants include tocopherols, dibutylhydroxytoluene, butylhydroxyanisole, and gallic acid esters. Examples of antioxidant acids include phosphoric acid, citric acid, ascorbic acid, maleic acid, malonic acid, succinic acid, lactic acid, fumaric acid, cephalin hexametaphosphate, phytic acid, and ethylenediamine tetraacetic acid. Mixtures of those materials may be used.

Various fragrances, fragrance components, and colorants may be included to provide desirable aesthetics for the compositions of the present invention.

### Personal care

Preferably as further ingredients personal care, in particular skin or body care compositions can include at least one of the following:
1) at least one additional surfactant, e.g. any surfactant mentioned above as surfactant (c) or (d)
2) waxes, such as e.g. carnuba, spermaceti, beeswax, lanolin, derivatives thereof as well as their mixtures;
3) plant extracts, for example vegetal oils such as avocado oil, olive oil, palm oil, palm nut oil, rape seed oil, linseed oil, soya oil, peanut oil, coriander oil, castor oil, poppy-seed oil, coconut oil, pumpkin seed oil, wheat germ oil, sesame oil, sunflower oil, almond oil, *macadamia* nut oil, apricot nut oil, hazel nut oil, jojoba oil or canola oil, aloe vera, camomile as well as their mixtures;
4) fatty compounds like
   - higher fatty acids such as lauric acid, myristic acid, palmitic acid, stearic acid, behenic acid, oleic acid, linoleic acid, linolenic acid, isostearic acid or polyunsaturated fatty acids;
   - higher fatty alcohols such as lauryl alcohol, cetyl alcohol, stearyl alcohol, oleyl alcohol, behenyl alcohol or 2-hexadecanol,
   - esters, such as cetyl octanoate, lauryl lactate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactates, alkyl citrates and/or alkyl tartrates;
   - lipids or phospholipids;
5) antioxidants;
6) moisturizing compounds;
7) vitamins such as vitamin A, B1, B2, B6, C and E and derivatives thereof; pantothenic acid and derivatives thereof; and biotin, and mixtures thereof, as well as vitamin alkyl esters;
8) anti-aging compounds;
9) sun protection agents / UV filter;
10) hydrocarbons such as paraffins, mineral oils, squalane or squalene;
11) preservatives;
12) complexing agents;
13) citric acid and/or citrate salt;
14) perfume.

To add at least any fragrance/perfume to the composition might be preferred to provide a pleasant odor.

### Hair care

If the composition is provided as a hair care composition, it might include (optionally in addition to any one of the above ingredients described for skin or body care) any active hair care ingredient, e.g.at least one selected from the group consisting of cationic polymers, cationic surfactants, cationic derivatized proteins, cationic derivatized protein hydrolysates and nonionic surfactant(s) (d), in particular fatty alcohols.

### Detergent

### Hard surface cleaning composition

If the composition is providing a detergent suitable for hard surface cleaning it is preferred that it comprises at least one of citric acid and/or citrate; nonionic surfactant(s) (d), in particular alkoxylated fatty alcohols, e,g, ethoxylated fatty alcohols; a bleaching system; enzyme(s).

### Laundry composition

If the composition is providing a detergent suitable for cleaning laundry it is preferred that it comprises at least one of anionic surfactant(s) (c), in particular sulfates or sulfonates as mentioned above; nonionic surfactant(s) (d), in particular alkoxylated fatty alcohols, e,g, ethoxylated fatty alcohols; enzyme(s); builder(s); complexing agent(s).

### Less preferred ingredients:

One idea underlying the invention was to provide effective personal care, hair care and cleaning compositions having less ingredients to minimize environmental and users burden, which in solid shape can be easy handled and transported, but provide a desired, also easy to handle aqueous composition when added to water. Therefore, several ingredients according to the present invention are limited in the solid and aqueous composition.

### Gelling or thickening agent(s)

It is preferred according to the invention that the composition comprises besides surfactant (a), (b) and optional (c) and / or (d) and/or (e) less than 5 wt.%, preferably less than 3 wt.%, more preferred less than 2 wt.%, even more preferred less than 1 wt.% or even less than 0,5 wt.% of any conventional gelling or thickening agent, selected from any type of polysaccharide or derivatives thereof, like cellulose, cellulose ester or cellulose ether, starch, starch ester or starch ether, agar-agar, xanthan, carrageen, pectine, alginate or similar; as well as from polyacrylic acid and any of its derivatives or copolymers.

The composition preferably comprises further less than 5 wt.%, preferably less than 3 wt.%, more preferred less than 2 wt.%, even more preferred less than 1 wt.% or even less than 0,5 wt.%, most preferred no glycol ester, glycol ethers, modified fatty compounds, hydrated castor oil, or inorganic salt(s) gelling or thickening agents.

Most preferred none of such additional conventional gelling or thickening agent(s) is/are present.

### Soap

The composition of the present invention can further comprise besides surfactants (a), (b) and optionally any of (c), (d) and (e) any soap, however preferably the composition comprises less than 20 wt.%, preferably less than 15 wt.%, more preferred less than 10 wt.%, even more preferred less than 5 wt.% and most preferred less than 3 wt.% of any soap, i.e. any fatty carboxylic acid salt. Most preferred the composition doesn't comprise any soap.

### Acyl-isethionate(s)

The composition according to any of the preceding aspects, can further comprise besides surfactant (a), (b) and optionally any of (c), (d) and (e) an acyl-isethionate, however, it is preferred that the composition comprises less than 5 wt.%, preferably less than 3 wt.%, more preferred less than 2 wt.%, even more preferred less than 1 wt.% or even less than 0,5 wt.% of any acyl-isethionate. Most preferred the composition doesn't comprise any acyl-isethionate. If any alkyl-isethionate is present, it is preferred that it is represented by methyl-isethionate (salt).

### Glycerol

The composition according to any of the preceding aspects, can further comprise glycerol, however, it is preferred that the composition comprises less than 5 wt.%, preferably less than 3 wt.%, more preferred less than 2 wt.%, even more preferred less than 1 wt.% or even less than 0,5 wt.% of glycerol. Most preferred the composition doesn't comprise glycerol.

### Silicone components

The composition according to any of the preceding aspects, can further comprise a silicone polymer or surfactant, however, it is preferred that the composition comprises less than 5 wt.%, preferably less than 3 wt.%, more preferred less than 2 wt.%, even more preferred less than 1 wt.% or even less than 0,5 wt.% of any silicone polymer or silicone surfactant. Most preferred the composition is free of those compounds.

### Package

The solid composition is provided preferably in a pre-dosed amount; thus, it can be provided in form of e.g. tablet(s) or powder or granulate portion(s). Said portions, in particular a powder or granulate can be provided packaged in any film or paper, wherein it is preferred to use biologically decomposable, non-plastic films or any paper packaging material. It is particularly preferred that the composition of the present invention comprises less than 5 wt.%, preferably less than 3 wt.%, more preferred less than 2 wt.%, even more preferred less than 1 wt.% or even less than 0,5 wt.% of polyvinyl alcohol (PVOH), most preferred no PVOH, which means neither included in the composition, nor as a wrapping film. The solid composition preferably is provided in a biologically decomposable film or in a paper bag.

The solid composition can be simply added to an appropriate amount of water to prepare an aqueous composition suitable for use as a cleaning composition for hard surfaces, like glass cleaning composition, dish cleaning composition, floor cleaning composition, bath cleaning composition, toilet cleaning composition, all-purpose cleaning composition, or as personal care composition, like a body cleansing composition, bath or shower gel, shampoo, body shrub, make up remover, face cleanser, or as a laundry composition, optionally dependent from the further ingredients of the solid composition.

To prepare one of these aqueous compositions an amount of the solid composition is combined with water so that 5 to 30 wt.% of said solid composition is added to an according amount of water to result in 100 wt.% of the aqueous composition. Accordingly, the aqueous composition comprises an amount of the formerly solid composition, dissolved in water, representing 5 to 30 wt.% of the aqueous composition. Preferably, an aqueous composition is obtained by combining 4 to 28 wt.%, 5 to 25 wt.%, 6 to 22 wt.%, 7 to 20 wt.%, 8 to 15 wt.%, 9 to 12 wt.% or 10 wt.% of the solid composition with water to a total amount of 100 wt.% of said aqueous composition.

As mentioned above it is particularly preferred that the solid composition of the present invention comprises or even is consisting of particles having a particle size in the range of from 0,01 µm to 3 mm, preferably from 0,05 µm to 2 mm, even more preferred from 0,1 to 1000 µm, including the lower limit of e.g. from 1 µm, 5 µm, 10 µm or 50 µm. It is preferred that less than 10 % by number of the particles have a particle size below the lower mentioned limit (defined e.g. by sieve analysis), more preferred less than 5 %, even more preferred less than 2 % or even less than 1 %, less than 0,5 % or less than 0,2 % by number. Further it is preferred that the particles have a maximum particle size of 3 mm, preferably 2 mm, more preferred 1 mm, which means that the powder doesn't comprise particles having a particle size above 3 mm, or above 2 mm or above 1 mm.

If this particle size range is used, the preparation of the aqueous composition with a desired viscosity is clearly faster and easier, because the particles readily and fully dissolve in water quickly enough to provide a homogenous aqueous composition, e.g. by just adding the solid composition to water, slewing, spinning, shaking or stirring the aqueous slurry a few seconds and allowing the container (bottle, beaker or any other suitable vessel) to stay e.g. 2 to 10 minutes to obtain the ready-prepared aqueous composition. If the particle size is too large, the particles, in particular the particles of the "gel-forming" acyl alkyl isethionate(s) (a), will form a jelly layer on the surface of the particles and therefore they cannot fully dissolve quickly. In this case it is necessary to spin, shake or stir the composition more intensively, which is not only time consuming, more effort, and the product cannot used immediately, but is also resulting in formation of foam, which can leak the container. It is particularly preferred that at least surfactant(s) (a) is present in particles having the above cited particle size distribution.

Particle sizes can be determined e.g. by dynamic or by static image analysis, for example with an image analysis type particle diameter distribution analyzer QICPIC/R¹⁶ (manufactured by Sympatec GmbH).

The solid composition according to the invention is used to prepare an aqueous, preferably liquid to gel-like composition. Due to the presence of surfactants of type (a) and (b) in a ratio as cited above in the aqueous composition it is not necessary to add additional rheology modifiers, gelling agents or thickening agents to obtain a liquid composition which is good to handle, easy to dose and suitable in application. Dependent from the amount of the solid composition added to water to prepare the aqueous composition the viscosity of the ready-prepared composition can vary, and it is possible to provide liquids having a dynamic viscosity in a range of from more than 1 mPa*s to 10.000 mPa*s, however, it is preferred that the ready-to-use aqueous composition has a viscosity in the range of from 4 mPa*s to less than 10.000 mPa*s at 20°C, preferably from 5 to 8.000, from 8 to 6.000, from 10 to 5.000, from 15 to 4.000 mPa*s at 20°C. For example a "thin" liquid composition like e.g. a face cleaning composition given on a cottage fleece before applied to the skin can have a viscosity of from e.g. 5 or 10 to less than 1.000, or from 12 to 800, from 15 to 700 or from 20 to 600, or from 30 to 550 mPa*s at 20°C (Brookfield viscosimeter e.g. LVDV2T Viscosimeter), spindle #4, 60 rpm), whereas a more "jellylike" liquid composition, e.g. a shampoo, bath or shower gel or a liquid laundry detergent can have a viscosity of from 1.000 to 6.000 mPa*s, or from 2.000 to 5.000 or from 2.500 to 4.000 mPa*s. All the values are given as measured with a rheometer at a temperature of 20°C using a Brookfield viscosimeter LVDV2T (spindle #4) at 60 revolutions per minute (RPM).

The aspects of the present invention are:
1. A solid composition comprising
   (a) at least one acyl alkyl isethionate surfactant and
   (b) at least one amphoteric surfactant
      in a ratio of (a) to (b) from 2:1 to 1:2,
      wherein each of said surfactants (a) and (b) is/are present in the solid composition in a total amount of from 20 to 60 wt.% based on the total amount of the solid composition, however, the total amount of said surfactants (a) and (b) together ranges from 45 to less than 100 wt.%.
2. The solid composition of aspect 1, wherein both surfactants (a) and (b) as such are solid at a temperature up to 40°C.
3. The solid composition of aspect 1 or 2, in tablet, powder or granulate form, preferably in powder or granulate form.
4. The composition of any of the preceding aspects, wherein at least surfactant(s) (a) is / are in solid powder form.
5. The composition of any of the preceding aspects, wherein surfactants (a) and (b) are both in solid powder form.
6. The composition of any of the preceding aspects, wherein the composition and/or the surfactant powder is represented by particles having a particle size in the range of from 0,01 µm to 3 mm, with a maximum particle size of 3 mm.
7. The composition of the aforesaid aspect, wherein the particles have a particle size in the range of from 0,05 µm to 2 mm, even more preferred from 0,1 to 1000 µm, including the lower limit of e.g. from 1 µm, 5 µm, 10 µm or 50 µm and a maximum particle size of 3 mm, preferably 2 mm, more preferred 1 mm.
8. The composition according to any of the preceding aspects, comprising less than 5 wt.%. preferably less than 3 wt.%. more preferred less than 2 wt.%. even more preferred less than 1 wt.% of water.
9. The composition of any of the preceding aspects, comprising less than 10 wt.%. preferably less than 8 wt.%. more preferred less than 5 wt.%. even more preferred less than 3 wt.% of any liquid component.
10. The composition of any of the preceding aspects, wherein surfactant (a) is a compound or a mixture of compounds according to formula R¹-COO-CR²R³-CR⁴R⁵-SO_{3⁻}-M⁺ wherein R¹ represents a C₄ to C₂₄ linear or branched alkyl group; R², R³, R⁴ and R⁵ independently represent a hydrogen atom or a C₁₋₄ alkyl group, wherein at least one of R², R³, R⁴ or R⁵ is not hydrogen, and M+ is a monovalent cation, preferably Na+, K+ monoethanolamine or ammonium ion.
11. The composition of the aforesaid aspect, wherein at least one of R² or R⁴ is a methyl group and the others of R², R³, R⁴ and R⁵ are hydrogen.
12. The composition of the aforesaid aspect, wherein surfactant(s) (a) is/are selected from sodium lauroyl methyl isethionate, sodium cocoyl methyl isethionate, sodium oleyl methyl isethionate and mixtures thereof.
13. The composition of the aforesaid aspect, wherein surfactant (a) comprises at least sodium lauroyl methyl isethionate, preferably is represented by sodium lauroyl methyl isethionate.
14. The composition of any of the preceding aspects, wherein surfactant(s) (b) is/are selected from at least one betaine, sultaine, amphodipropionate or amphoacetate.
15. The composition of the aforesaid aspect, wherein surfactant (b) is selected from cocamidopropyl betaine, lauramidopropyl betaine, coamidopropyl hydroxy sultaine, disodium cocoamphodipropionate or sodium lauroamphoacetate, or a mixture thereof.
16. The composition of the aforesaid aspect, wherein surfactant (b) comprises at least cocamidopropyl betaine, preferably is represented by cocamidopropyl betaine.
17. The composition of any of the preceding aspects, wherein surfactant(s) (a) is / are present in a total amount of from 22 wt.% to 50 wt.%, preferably in a total amount of from 25 wt.% to 40 wt.% based on the total amount of the solid composition.
18. The composition of any of the preceding aspects, wherein surfactant(s) (b) is /are present in a total amount of from 22 wt.% to 50 wt.%, preferably in a total amount of from 25 wt.% to 40 wt.% based on the total amount of the solid composition.
19. The composition of any of the preceding aspects, wherein the ratio of surfactant (a) to (b) is in the range of from 1,8:1 to 1:1,8, in the range of from 1,6:1 to 1:1,6, in the range of from 1,5:1 to 1:1,5, in the range of from 1,4:1 to 1:1,4, in the range of from 1,3:1 to 1:1,3, in the range of from 1,2:1 to 1:1,2, in the range of from 1,1:1 to 1:1,1 or in the ratio of 1:1.
20. The composition of any of the preceding aspects, comprising further at least one additional anionic surfactant (c), selected from sulfate, sulfonate, sulfoacetate, sulfosuccinate, or carbonate surfactants, in particular alkyl sulfate, alkyl sulfonate, alkyl benzene sulfonate, alkyl sulfoacetate, alkyl sulfosuccinate, alkyl carboxylate (i.e. soap) surfactants.
21. The composition of the aforesaid aspect, comprising a C₆₋₂₄ alkyl chain, more preferred a C₈₋₂₂ alkyl chain, even more preferred a C₁₀₋₂₀ alkyl chain and even more preferred a C₁₂₋₁₈ alkyl chain, or a mixture of surfactants as defined before, having different alkyl chain length.
22. The composition of any of the preceding aspects, comprising as component (c) at least one C₁₂₋₁₈ alkyl sulfate ester or a mixture of C₁₂₋₁₈ alkyl sulfate esters, like e.g. sodium coco sulfate or palm kernel oil sulfate.
23. The composition of any of the preceding aspects, comprising as component (c) at least one C₁₂₋₁₈ alkyl sulfoacetate, e.g. sodium lauryl sulfo acetate.
24. The composition of any of the preceding aspects, comprising as component (c) at least one C₁₂₋₁₈ alkyl glutamate, e.g. sodium cocoyl glutamate.
25. The composition of any of the preceding aspects, comprising as component (c) at least one C₁₂₋₁₈ alkyl isethionate, e.g. sodium cocoyl isethionate.
26. The composition of any of the preceding aspects, wherein surfactant(s) (c) is/are present in a total amount of from 0,2 to 10 wt.% of the total solid composition, preferably in an amount of from 0,5 to 8 wt.%, more preferred in an amount of from 1 to 6 wt.%.
27. The composition of any of the preceding aspects, wherein the counter-ion in surfactant(s) (c) is selected from Na+, K+ monoethanolamine or ammonium ion.
28. The composition of any of the preceding aspects, comprising further citric acid and/or citrate salt.
29. The composition according to the aforesaid aspect, wherein
   (i) citric acid is present in an amount of from 0,5 to 20 wt.% of the total solid composition, preferably in an amount of from 1 to 15 wt.%, more preferred from 2 to 10 wt.% even more preferred from 3 to 8 or from 4 to 6 wt.% of the total solid composition and/or
   (ii) citrate salt is present in an amount of from 0,5 to 20 wt.% of the total solid composition, preferably in an amount of from 1 to 15 wt.%, more preferred from 2 to 10 wt.% even more preferred from 3 to 8 wt.% or from 4 to 6 wt.% of the total solid composition.
30. The composition according to the aforesaid aspect, wherein the total amount of a combination of citric acid and citrate salt in the total solid composition ranges from 1 to 30 wt.%, preferably 2 to 25 wt.%, more preferred 4 to 20 wt.%, even more preferred 5 to 15 wt.% or 6 to 10 wt.% of the total solid composition.
31. The composition of any of the preceding aspects, comprising further any additional α-hydroxy acid like e.g. lactic acid, maleic acid, glycolic acid, mandelic acid, tartaric acid, or isocitric acid.
32. The composition according to the aforesaid aspect, wherein the total amount of the additional α-hydroxy acid(s) - except citric acid and citrate salt - in the total solid composition ranges from 0,5 to 30 wt.%, preferably 1 to 25 wt.%, more preferred 2 to 20 wt.%, even more preferred 2,5 to 15 wt.% or 3 to 10 wt.% of the total solid composition.
33. The composition of any of the preceding aspects, comprising further a preservative agent, preferably selected from benzoic acid, benzoates like e.g. sodium benzoate, sorbates like e.g. potassium sorbate, sulfites, phenoxyethanol, sodium and zinc pyrithione, bronopol, iodopropynylbutylcarbamate, formaldehyde and formaldehyde releaser, glyoxal, glutaral and isothiazolinones like e.g. methylisothiazolinone, chloromethylisothiazolinone, benzisothiazolinone, octylisothiazolinone, and butylbenzisothiazolinone.
34. The composition according to the aforesaid aspect, wherein at least a benzoate or a sorbate is present as at least one preservative agent, or both are present in the solid composition.
35. The composition according to the preceding aspects, wherein each of the preservative agents can independently be present in an amount of from 0,2 to 10 wt.% of the total solid composition, preferably in an amount of from 0,5 to 8 wt.%, more preferred from 0,8 to 6 wt.% even more preferred from 1 to 5 wt.% of the total solid composition.
36. The composition according to the aforesaid aspect, wherein the total amount of preservative agents in the solid composition is 0 to 15 wt.%, preferably 1 to 12 wt.%, 2 to 10 wt.% or 3 to 8 wt.% of the total composition.
37. The composition of any of the preceding aspects, comprising further a complexing agent separate from citric acid or citrate.
38. The composition according to the aforesaid aspect, wherein said complexing agent is selected from MGDA or GLDA, ethylenediamine-N,N'-disuccinic acid, imminodisuccinic acid, diethylene triamine pentaacetic acid, ethylenediamine tetraacetic acid, (polymeric) phosphonate(s), e.g. diethylenetriamine pentamethylene phophonate, tetrahydroxypropyl ethylenediamine, triisobutylphosphate, EDTA, dicarboxymethyl alaninate, etidronate, polyphosphate, maleic acid/acrylic acid copolymer(s), each of them preferably in form of their sodium salts.
39. The composition according to any of the preceding aspects, comprising further a total amount of 0 to 60 wt.% of at least one skin compatible humidity-uptaking (hygroscopic) compound, in particular any of C4- C5- or C6-sugars or dimers or oligomers thereof, preferably glucose, fructose, saccharose, sorbitol, erythritol, or any hygroscopic salt like e.g. sodium chloride or sodium sulfate.
40. The composition according to the aforesaid aspect, wherein each of the at least one humidity-uptaking compound, is independently present in an amount of from 0 to 30 wt.%, preferably 1 to 20 wt.%, more preferred 2 to 10 wt.%.
41. The composition of any of the preceding aspects, comprising further at least one body care, skin care or hair care compound.
42. The composition according to the aforesaid aspect, wherein said skin care or body care compound is selected from fatty compound(s), oil, wax, anti-aging compound(s), antioxidant(s), moisturizer(s), vitamin(s), wherein said hair care compound is selected from cationic polymers, nonionic or cationic surfactants, cationic derivatized proteins, cationic derivatized protein hydrolysates and fatty alcohols.
43. The composition according to any of the preceding aspects, comprising
   besides surfactant (a), (b) and optional (c) less than 5 wt.%, preferably less than 3 wt.%, more preferred less than 2 wt.%, even more preferred less than 1 wt.% or even less than 0,5 wt.% of any gelling or thickening agent, selected from any type of polysaccharide or derivatives thereof, like cellulose, cellulose ester or cellulose ether, starch, starch ester or starch ether, agar-agar, xanthan, carrageen, pectine, alginate or similar; as well as from polyacrylic acid and any of its derivatives or copolymers, most preferred no such gelling or thickening agent is present.
44. The composition according to the aforesaid aspect, wherein further less than 5 wt.%, preferably less than 3 wt.%, more preferred less than 2 wt.%, even more preferred less than 1 wt.% or even less than 0,5 wt.%, most preferred no glycol ester, glycol ethers, modified fatty compounds, hydrated castor oil, or inorganic salt(s) is contained.
45. The composition according to any of the preceding aspects, comprising
   besides surfactant (a) and (b) less than 20 wt.%, preferably less than 15 wt.%, more preferred less than 10 wt.%, even more preferred less than 5 wt.% and most preferred less than 3 wt.% of any soap.
46. The composition according to any of the preceding aspects, comprising
   besides surfactant (a), (b) and optional (c) less than 5 wt.%, preferably less than 3 wt.%, more preferred less than 2 wt.%, even more preferred less than 1 wt.% or even less than 0,5 wt.% of any acyl-isethionate.
47. The composition according to any of the preceding aspects, comprising
   less than 5 wt.%, preferably less than 3 wt.%, more preferred less than 2 wt.%, even more preferred less than 1 wt.% or even less than 0,5 wt.%, most preferred no polyvinyl alcohol, neither included in the composition, nor as a wrapping film.
48. The composition according to any of the preceding aspects, comprising
   less than 5 wt.%, preferably less than 3 wt.%, more preferred less than 2 wt.%, even more preferred less than 1 wt.% or even less than 0,5 wt.%, most preferred no glycerol.
49. The composition according to any of the preceding aspects, comprising
   less than 5 wt.%, preferably less than 3 wt.%, more preferred less than 2 wt.%, even more preferred less than 1 wt.% or even less than 0,5 wt.%, most preferred no silicone polymer or silicone surfactant.
50. The composition according to any of the preceding aspects, provided in form of pre-dosed powder or granulate portions.
51. The composition according to any of the preceding aspects, wherein said composition is composed of particles having a particle size as defined in aspects 6 or 7.
52. An aqueous composition obtained by combining a solid composition of any of the preceding aspects with water, wherein said aqueous composition comprises 3 to 30 wt.% of said (formerly) solid composition according to any of the preceding aspects in water.
53. An aqueous composition according to the aforementioned aspect, obtained by combining 4 to 28 wt.%, 5 to 25 wt.%, 6 to 22 wt.%, 7 to 20 wt.%, 8 to 15 wt.%, 9 to 12 wt.% or 10 wt.% of the solid composition of any of the preceding aspects with water to a total amount of 100 wt.%.
54. An aqueous composition according to the preceding aspects, said aqueous composition has a dynamic viscosity of from 4 to 10.000 mPa*s at 20°C, e.g. 5 mPa*s to 8.000 mPa*s at 20°C, preferably from 8 to 6.000, from 10 to 5.000, from 15 to 4.000 mPa*s at 20°C.
55. The aqueous composition of any of the preceding aspects, wherein said composition is a cleaning composition for hard surfaces, like glass cleaning composition, floor cleaning composition, bath cleaning composition, toilet cleaning composition, all-purpose cleaning composition, or a personal care cleaning composition, like a body cleansing composition, bath or shower gel, shampoo, body shrub, make up remover, face cleanser, or a laundry composition.
56. An aqueous composition according to the aforementioned aspect, wherein
   (i) said solid composition comprises citric acid or citrate according to the aspects as cited above, if it is provided as a hard surface cleaning composition,
   (ii) said solid composition comprises a hair, skin or body care component, if it is provided as a personal care cleaning composition,
   (iii) said solid composition comprises an additional anionic surfactant (c), preferably alkyl benzene sulfonate or alkyl benzene sulfonic acid, if it is provided as a laundry detergent.
57. Use of an acyl alkyl isethionate as defined in any of the preceding aspects as surfactant (a) as a rheology modifying agent, preferably as a thickening agent in an aqueous composition.
58. Use of a combination of at least one acyl alkyl isethionate as defined in any of the preceding aspects being surfactant (a) and at least one amphoteric surfactant as defined in any of the preceding aspects being surfactant (b) for modifying rheology of an aqueous composition.
59. Use according to the aforementioned aspect, wherein said acyl alkyl isethionate (a) and said amphoteric surfactant (b) are used in a weight ratio of from 2 : 1 to 1 : 2 or in a weight ratio as defined in aspect 19.
60. Use according to the aforementioned aspect, wherein surfactants (a) and (b) are used in water or an aqueous composition in an amount resulting in a viscosity of from 10 to 10.000 mPa*s, wherein preferably no further rheology modifying, thickening or gelling agent is present in the composition.
61. Use according to any of aspects 57 to 60, wherein the ready-to-use aqueous composition has a viscosity in the range of from 10 mPa*s to less than 10.000 mPa*s at 20°C, preferably from 15 to 8.000, from 20 to 6.000, from 30 to 5.000, from 50 to 4.000 mPa*s at 20°C.
62. Use according to the aforementioned aspect, wherein the aqueous composition has a viscosity of from 5 or 10 to less than 1.000, or from 12 to 800, from 15 to 700 or from 20 to 600, or from 30 to 550 mPa*s at 20°C (Brookfield viscosimeter e.g. LVDV2T Viscosimeter), spindle #4, 60 rpm), or it has a viscosity of from 1.000 to 6.000 mPa*s, or from 2.000 to 5.000 or from 2.500 to 4.000 mPa*s.

### Examples

Commercially available flakes of sodium lauroyl methyl isethionate (ISELUX^{®} available from Innospec, Germany) have been pulverized in a pulverisator, and optionally sieved to obtain a powder having a particle size in the range of from 0.1 to 3000 µm. Cocamidopropyl betaine is commercially available in powder form with particle sizes below 3 mm. Optionally said powder can be sieved as well before using in the solid composition according to the invention.

Further ingredients for the solid compositions are combined in suitable amounts for preparing a "base powder composition":
Alkylsulfate(s) as further anionic surfactant(s), citric acid/citrate, preservative(s), hygroscopic agent(s), complexing agent, NaCl, perfume, pH adjusting agent and cationic polymer.

In the following exemplifying solid compositions said "base composition" was combined with the powdered sodium lauroyl methyl isethionate as surfactant (a) and cocamidopropyl betaine as surfactant (b) in the following ratios, mixed until representing a homogenous mixture and added to water (10 wt.% respectively of the solid composition ad 100 wt.% with tap water (20°C)). The aqueous composition was stirred at 100 rpm with a stirrer for 2 min, allowed to stand for 10 min, then viscosity was measured at room temperature (20°C) with a Brookfield viscosimeter LVDV2T, spindle #4 at 60 rpm. Thereafter the aqueous composition was allowed to stand for further 5 days at room temperature before viscosity again was determined under the same conditions.

**Table 1**

| Ingredient | composition 1 | composition 2 | composition 3 | composition 4 | composition 5 |
|---|---|---|---|---|---|
| Surfactant (a) | 52 wt.% | 43,3 wt.% | 32,5 wt.% | 21,7 wt.% | 20 wt.% |
| Surfactant (b) | 13 wt.% | 21,7 wt.% | 32,5 wt.% | 43,3 wt.% | 45 wt.% |
| Base powder | 35 wt.% | 35 wt.% | 35 wt.% | 35 wt.% | 35 wt. % |
| Ratio (a) : (b) | 4:1 | 2:1 | 1:1 | 1:2 | 1:2,3 |
| Viscosity after preparation | about 1 mPa*s | 530 mPa*s | 2.800 mPa*s | 3.800 mPa*s | 3.900 mPa*s |
| Viscosity after 5 days | about 1 mPa*s | 500 mPa*s | 2.500 mPa*s | 3.700 mPa*s | 4.200 mPa*s |
| Appearance after 5 day * | A | B | C | D | E |

| | | | | | |
|---|---|---|---|---|---|
| * Rating of appearance: A = transparent homogenous "water-like" liquid; B = transparent homogenous soft-thickened liquid; C = homogenous soft-jelly liquid; D = homogenous soft gel; E = streaky gel | | | | | |

As can be seen in the present examples, the combination of a surfactant of type (a) with a surfactant of type (b) in a ratio within the range of from 2:1 to 1:2 results in liquid to jelly aqueous compositions which have a suitable viscosity to be used e.g. as a shampoo, shower gel or liquid detergent, whereas a ratio of 4:1 results in "water-thin" solutions, not suitable to be handled by e.g. pouring in the users hand. Further, compositions 2, 3 and 4 are storage-stable concerning the viscosity, wherein viscosity decreases a little bit during storage, but still remains in a range providing a ready-prepared product which is easy to handle, to pour and to apply e.g. on the skin or hair of the user. By increasing the amount of surfactant (b) to a ratio of surfactant (a) : (b) beyond 1:2 (as in composition 5), the viscosity during storage increases, which is not desired, resulting in a streaky, not fully homogenous gel.

## Claims

1. A solid composition comprising
(a) at least one acyl alkyl isethionate surfactant and
(b) at least one amphoteric surfactant
in a ratio of (a) to (b) from 2:1 to 1:2,
wherein each of said surfactants (a) and (b) is/are present in the solid composition in a total amount of from 20 to 60 wt.% based on the total amount of the solid composition, however, the total amount of said surfactants (a) and (b) together ranges from 45 to less than 100 wt.%.

2. The composition of claim 1, wherein surfactant (a) is a compound or a mixture of compounds according to formula R¹-COO-CR²R³-CR⁴R⁵-SO₃⁻M⁺ wherein R¹ represents a C₄ to C₂₄ linear or branched alkyl group; R², R³, R⁴ and R⁵ independently represent a hydrogen atom or a C₁₋₄ alkyl group, wherein at least one of R², R³, R⁴ or R⁵ is not hydrogen, and M+ is a monovalent cation, preferably Na+, K+ monoethanolamine or ammonium ion.

3. The composition of the previous claim, wherein surfactant(s) (a) is/are selected from sodium lauroyl methyl isethionate, sodium cocoyl methyl isethionate, sodium oleyl methyl isethionate and mixtures thereof, wherein preferably surfactant (a) comprises at least sodium lauroyl methyl isethionate, more preferred is represented by sodium lauroyl methyl isethionate.

4. The composition of any of the preceding claims, wherein surfactant(s) (b) is/are selected from at least one betaine, sultaine, amphodipropionate or amphoacetate, preferably surfactant (b) is selected from cocamidopropyl betaine, lauramidopropyl betaine, coamidopropyl hydroxy sultaine, disodium cocoamphodipropionate or sodium lauroamphoacetate, or a mixture thereof, more preferred surfactant (b) comprises at least cocamidopropyl betaine, preferably is represented by cocamidopropyl betaine.

5. The composition of any of the preceding claims, wherein surfactant(s) (a) and surfactant(s) (b) each independently are present in a respective total amount of from 22 wt.% to 50 wt.%, preferably in a total amount of from 25 wt.% to 40 wt.% based on the total amount of the solid composition.

6. The composition of any of the preceding claims, wherein the ratio of surfactant(s) (a) to (b) is in the range of from 1,8:1 to 1:1,8, in the range of from 1,6:1 to 1:1,6, in the range of from 1,5:1 to 1:1,5, in the range of from 1,4:1 to 1:1,4, in the range of from 1,3:1 to 1:1,3, in the range of from 1,2:1 to 1:1,2, in the range of from 1,1:1 to 1:1,1 or in the ratio of 1:1.

7. The composition of any of the preceding claims, comprising further at least one additional anionic surfactant (c), selected from sulfate, sulfonate, sulfoacetate, sulfosuccinate, or carbonate surfactants, in particular alkyl sulfate, alkyl sulfonate, alkyl benzene sulfonate, alkyl sulfoacetate, alkyl sulfosuccinate surfactants, preferably comprising a C₆₋₂₄ alkyl chain, more preferred a C₈₋₂₂ alkyl chain, even more preferred a C₁₀₋₂₀ alkyl chain and even more preferred a C₁₂₋₁₈ alkyl chain, or a mixture of surfactants as defined before, having different alkyl chain length.

8. The composition of any of the preceding claims, comprising further at least one body care, skin care or hair care compound, wherein preferably said skin care or body care compound is selected from fatty compound(s), oil, wax, anti-aging compound(s), antioxidant(s), moisturizer(s), vitamin(s), wherein said hair care compound is selected from cationic polymers, nonionic or cationic surfactants, cationic derivatized proteins, cationic derivatized protein hydrolysates and fatty alcohols.

9. The composition according to the previous claim, wherein the composition comprises, consists of or is made of ingredients in particle form, wherein at least surfactant(s) (a) have a particle size in the range of from 0,01 µm to 3 mm, with a maximum particle size of 3 mm, preferably at least surfactants (a) and (b), even more preferred the whole composition has a particle size in in the range of from 0,01 µm to 3 mm, with a maximum particle size of 3 mm.

10. The composition according to any of the preceding claims comprising
besides surfactants (a), (b) and optional (c) less than 5 wt.%, preferably less than 3 wt.%, more preferred less than 2 wt.%, even more preferred less than 1 wt.% or even less than 0,5 wt.% of any gelling or thickening agent selected from any type of polysaccharide or derivatives thereof, like cellulose, cellulose ester or cellulose ether, starch, starch ester or starch ether, agar-agar, xanthan, carrageen, pectine, alginate or similar; as well as polyacrylic acid and any of its derivatives or copolymers, most preferred no such additional gelling or thickening agent.

11. The composition according to the previous claim, wherein further less than 5 wt.%, preferably less than 3 wt.%, more preferred less than 2 wt.%, even more preferred less than 1 wt.% or even less than 0,5 wt.%, most preferred no glycol ester, glycol ethers, modified fatty compounds, hydrated castor oil, or inorganic salt(s) is contained.

12. The composition according to any of the preceding claims, provided in form of a tablet or of pre-dosed powder or granulate portions, preferably in form of pre-dosed powder or granulate portions.

13. An aqueous composition obtained by combining a solid composition of any of the preceding claims with water, wherein said aqueous composition comprises 3 to 30 wt.% of said (formerly) solid composition according to any of the preceding aspects in water.

14. The aqueous composition according to the previous claim, wherein said composition is a cleaning composition for hard surfaces, like glass cleaning composition, floor cleaning composition, bath cleaning composition, toilet cleaning composition, all-purpose cleaning composition, or a personal care cleaning composition, like a body cleansing composition, bath or shower gel, shampoo, body shrub, make up remover, face cleanser, or a laundry composition.

15. Use of a combination of an acyl alkyl isethionate as defined in the preceding claims as surfactant (a) and an amphoteric surfactant defined in the preceding claims as surfactant (b) as rheology modifying agents, preferably as a thickening agent is an aqueous composition, wherein preferably said surfactants are used in water or an aqueous composition in an amount resulting in a viscosity of from 10 to 10.000 mPa*s, wherein further preferred no further rheology modifying, thickening or gelling agent is present in the composition.
